# EUROPEAN PATENT SPECIFICATION

(11) **EP 1 353 602 B1**
(45) Date of publication and mention of the grant of the patent: **09.11.2005**
(21) Application number: 02715939.1
(22) Date of filing: 22.01.2002
(51) Int. Cl.: A61N 7/02

(54) **APPARATUS FOR HIGH ENERGETIC ULTRASONIC TISSUE TREATMENT**
VORRICHTUNG ZUR BEHANDLUNG VON GEWEBE MIT ULTRASCHALL HOHER ENERGIE
APPAREIL DE TRAITEMENT DE TISSU PAR ULTRASONS A HAUTE ENERGIE

(30) Priority: 22.01.2001 SE 0100160
(43) Date of publication of application: 22.10.2003
(73) Proprietor: ATOS MEDICAL AB, 242 22 Hörby (SE)
(72) Inventor: HOLMER, Nils-Gunnar, S-226 47 Lund (SE)
(74) Representative: Petri, Stellan
(86) International application number: PCT/SE2002/000099
(87) International publication number: WO 2002/056780

(56) References cited:
- EP-A- 0 659 387
- US-A- 5 938 608
- US-A- 6 132 378
- PATENT ABSTRACTS OF JAPAN vol. 1998, no. 14, 31 December 1998 (1998-12-31) & JP 10 248850 A (OLYMPUS OPTICAL CO LTD), 22 September 1998 (1998-09-22)

## Description

### Background of the invention

### Field of the invention

The invention relates to apparatus for high energetic ultrasonic tissue shrinkage in a target area inside a living body from the outside or a cavity of the living body.

More particularly the invention relates to apparatus for eliminating or substantially reducing snoring and opening up or enlarging narrow airways and for tissue shrinkage in the head and neck region by non-invasive ultrasonic medical treatment.

### Description of the Prior Art

Snoring is the result of several contributing factors such as narrow airways, enlarged tongue, deviated nasal septum, and enlarged turbinates or nasal polyps. Another reason can be a decrease in upper airway muscle tone, occurring during sleep. These conditions produce an increased airway resistance and a negative intraluminal pressure during inspiration resulting in traction and vibration of tissues in the upper airway. Most prone to vibrate is the soft tissue, including the tonsils, soft palate and uvula but also the tongue base. Snoring is associated with serious health risks. It may result in significant sleep disruption or fragmentation that may lead to daytime fatigue and sleepiness resulting in safety risks. It has been shown that habitual snoring is related to hypertension, hearts disease and stroke. During pregnancy snoring is a significant health risk both for the woman and for the child. Another serious condition associated with snoring is obstructive sleep apnea which leads to increased mortality.

Several methods to treat snoring have been developed. Conservative, mechanical and pharmacological non-surgical methods such as weight reduction, alteration of sleep position, smoking cessation, and elimination of sedatives or alcohol intake during evening hours. Dental prosthetic and tongue retaining apparatuses are used with positive effect in some selective patients. Medical treatment of nasal allergies and endocrine problems can be helpful for certain patients.

However, when snoring becomes loud, habitual and disruptive to others, the patients ought to be considered for a more advanced intervention. Uvulopalathopharyngoplasty (UPPP) is a conventional surgical method demanding general anesthesia which can be difficult in this group of patients with narrow airways. The method of choice today is laser-assisted uvulopalatoplasty (LAUP). In local anesthesia the soft palate (velum palatinum) and the uvula are vaporized and ablated using a CO₂-laser equipment. This type of operation can be performed in small offices as a single operation or sometimes have to be done in as much as 5 different stages. One drawback of this method is that tissues are actually cut away during the operation and that the risk for infection therefore cannot be excluded. Accordingly, the patients are treated with antibiotics postoperatively. Another major drawback is the heavy pain during several weeks with need for systematic pain medication and topical anesthetics. The patients are often unable to work for about two weeks after the operation.

Another invasive method to treat snoring is to insert RF-electrodes as needles in the tissue to be treated (e.g. tongue, uvula, soft palate, turbinates). Through the needles electromagnetic energy is delivered which will diminish the tissue by heating.

JP-A-05076538 describes an ultrasonic therapeutic apparatus for treating deceased tissue by heating with ultrasonic energy so as to destroy the deceased tissue, wherein means are provided for detecting the position of the target area and for focusing the ultrasonic energy on that area. A transducer for emitting ultrasound energy from an ultrasound generator is displaceably mounted in a water filled housing partly defined by a membrane which is engaged with the skin or mucous membrane at the treatment site. The position of the transducer is adjusted in relation to the housing and thus in relation to the membrane in order to accurately focus the emitted ultrasound energy on the target area to be treated so that an accurate treatment of the target area will be achieved while avoiding detrimental influence on surrounding tissue as well as skin and mucous membrane.

US-A-4 936 303 discloses a similar apparatus having a housing with a membrane which is engaged with the skin at the treatment site. In this case fluid is circulated through the interior of the housing over the inside surface of the membrane said fluid being temperature controlled to provide a constant surface temperature at the treatment site.

An apparatus according to the preamble of claim 1 is disclosed in EP-A- 0 659 387.

### Brief Summary of the Invention

The primary object of the present invention is to provide apparatus for high energetic ultrasonic tissue shrinkage in a target area inside a living body from an outside surface or a cavity of the living body in order to achieve a fibrous tissue development and thereby shrinkage of the tissue under mucous membrane or skin in the head and neck region without destroying or adversely affecting tissue surrounding the target area.

Said object is achieved according to the invention by an apparatus of the kind disclosed in EP-A-0 659 387 having the characterising features according to claim 1.

Another object of the invention is to provide an apparatus of the kind referred to which produces and controls cooling of the skin or mucous membrane at the treatment site.

A further object of the invention is to provide an apparatus of the kind referred to which is particularly well suited for treatment of the uvula and the soft palate adjacent the uvula at each side thereof in order to eliminate or substantially reduce snoring. By exposure of the uvula and the soft palate to high energetic ultrasound fibrous tissue development will be achieved and thereby shrinkage of the tissue under the mucous membrane, which has a comparable effect as removal of said tissue by surgery (UPPP or LAUP) since the vibrating tissue mass will be reduced.

A still further object of the invention is to provide an apparatus of the kind referred to which allows focusing of the ultrasound energy to be adjusted in dependence of the location of the target area so that the ultrasound energy always will be focused on the target area.

In order to effect the treatment in a user friendly manner the exchangeable unit provides both a sterility barrier and a contact surface cooling the treatment site.

The diagnostic part of the invention has also the possibility of controlling the result after each therapeutic pulse, in order to produce a well adjusted energy dose for creating shrinkage of the targeted tissue. The controlling system analyses the backscattered ultrasound signals from the area around the focus point. The analysis can be based on the amount of backscattered harmonics, Dopplershift and the difference between echoes from positive and negative pulses of either diagnostic or therapeutic ultrasound energy from untreated tissue.

The further objects mentioned above are achieved by preferred embodiments of the invention defined in the dependent claims.

The invention will be described in more detail below with reference to the accompanying drawings showing illustrative embodiments of the invention.

### Brief Description of the Drawing

**FIG 1** is a side view of a first embodiment of the apparatus of the invention,
**FIG 2** is a plan view of an instrument forming part of the apparatus in FIG 1 to be held by the operator the instrument being shown in a straight condition,
**FIG 3** is a plan view of the instrument in FIG 2 in a bent condition,
**FIG 4** is a partial side view of the instrument in FIGS 2 and 3, which is intended for multiple use,
**FIG 5** is a side view of the part of the instrument shown in FIG 4 with an element intended for one way use mounted thereon,
**FIG 6** is an end view of the instrument,
**FIG 7** is a cross sectional view taken along line A-A in FIG 6,
**FIG 8** is a cross sectional view taken along line B-B in FIG 6,
**FIG 9** is a cross sectional view taken along line C-C in FIG 6,
**FIG 10** is a partial cross sectional view according to FIG 7 showing the instrument in a different adjusted position than that in FIG 7,
**FIG 11** is a side view of a second embodiment of the instrument of the invention,
**FIG 12** is a plan view of the instrument in FIG 11,
**FIG 13** is an enlarged cross sectional view taken along line
   D-D in FIG 11,
**FIG 14** is an enlarged cross sectional view taken along line E-E in FIG 12,
**FIG 15** discloses different shapes of the transducer head in side view, and
**FIG 16** is a block diagram of a control unit forming part of the apparatus of the invention, and
**FIG 17** is a time diagram of the procedure applied when using the apparatus,

### Detailed Description of the Invention

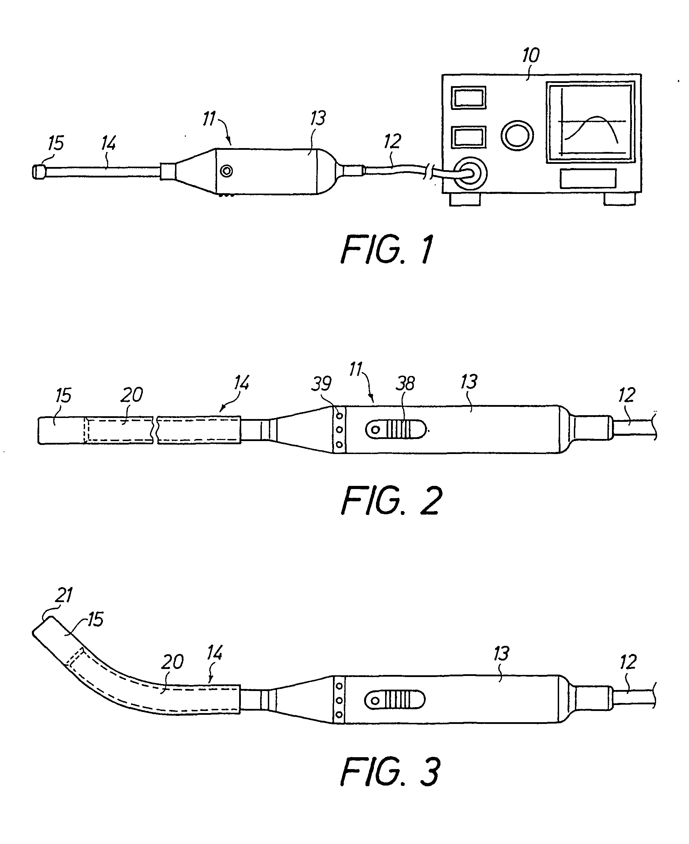

The apparatus of the invention disclosed in FIGS 1 to 3 comprises a control unit 10 and an instrument 11 to be held by the operator, which is connected with the control unit by a flexible hose 12 containing electric wiring and fluid conduits. The instrument forms a handle 13 and a stem 14 projecting from the handle. An ultrasonic transducer head 15 is provided at the free end of the stem, facing axially from the end.

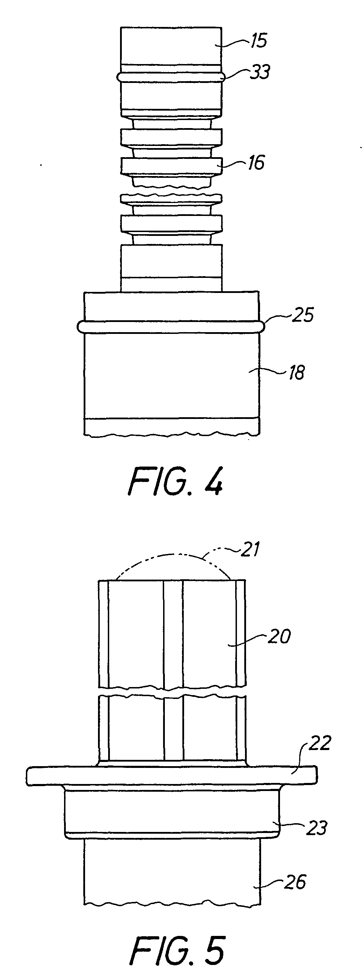

Referring also to FIGS. 4 to 10 the stem 14 comprises a series of individual elements 16 which are kept in mutual engagement at concave and convex surfaces by a helical tension spring 17 extending through the elements and being attached at the ends of the spring to end elements 16A and 16B. End element 16A is connected with transducer head 15 while end element 16B is connected with a bushing 18 which has outside threads 19 and is formed integral with or is attached to the handle 13.

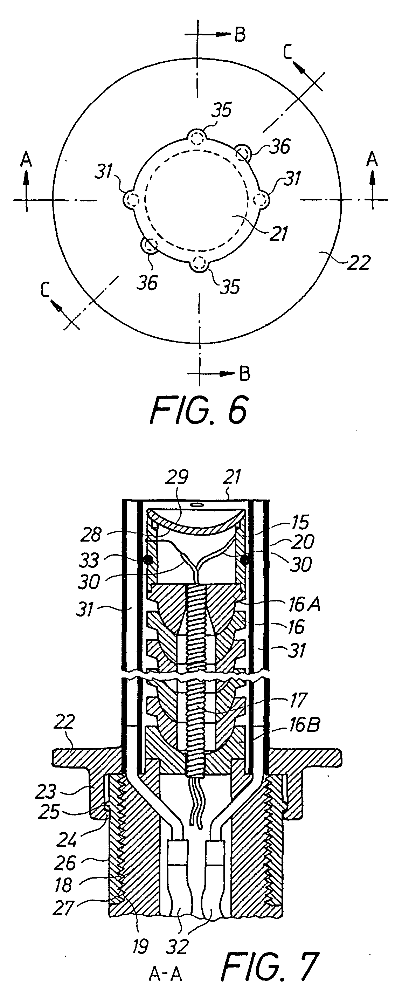

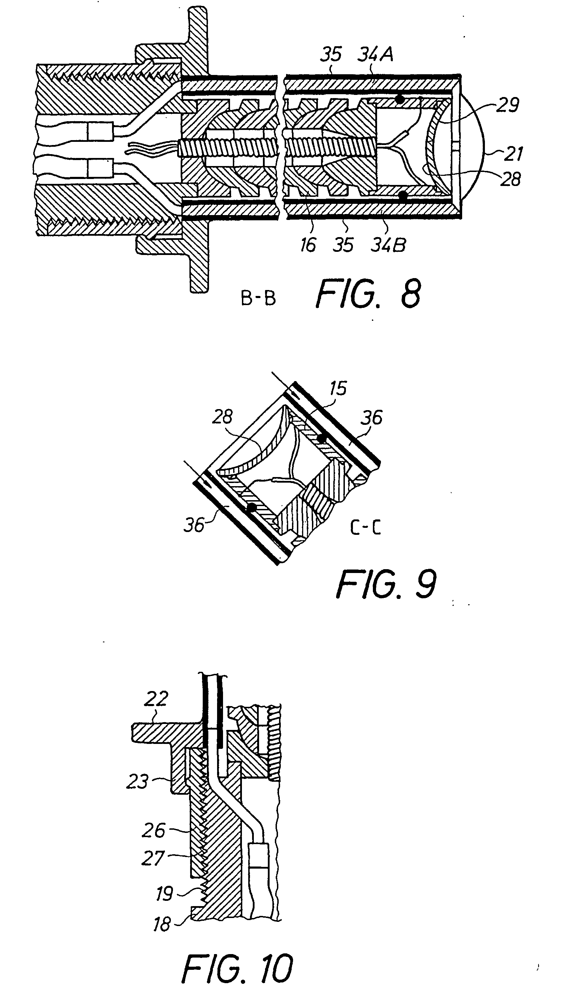

An open-ended socket 20 is closed at one end by a flexible and resilient membrane 21 to be applied against a surface of the human body. At the other end socket 20 is connected to a flange 22. A collar 23 integral with the flange forms an inside annular bead 24 which is snapped over an outside annular bead 25 on a socket 26. Thus, socket 20 with membrane 21 can be separated from bushing 18 in order to be thrown away after use or be sterilized before it is used again. Socket 26 has inside threads 27 engaging the outside threads 19 of bushing 18. Transducer head 15 comprises a piezo-electric crystal 28 forming a concave surface 29 which faces membrane 21 and is connected with two wires 30 which are extended through spring 17 and hose 12 to control unit 10 for the supply of electric current exciting crystal 28. Socket 20 forms two axial passages 31, FIGS 6 and 7, connected to hoses 32 which are extended through hose 12 to control unit 10. A fluid, water or air, can be circulated through passages 31 and the space defined by the concave surface 29 and membrane 21 in order to cool the crystal and the membrane as well as the body surface against which the membrane is applied during operation of the apparatus, but also to expand membrane 21 (see FIG 8) for adjustment of the distance between the crystal and said body in order to focus the ultrasound energy emitted by the crystal, on the target area to be treated in the human body. Adjustment of the distance between the crystal and the body surface is effected by varying the pressure of the circulating fluid. An O-ring 33 seals said space defined by the concave surface 29 and the membrane 21, against the interior of the stem. Optical fibers 34A and 34B are extended through axial passages 35 formed by socket 20, FIGS 6 and 8, and through hose 12 to transmit to the control unit 10 signals representative for the temperature of the membrane 21. Fiber 34A projects light against the back surface of the membrane 21, which can be covered by a temperature sensitive paint that changes color in dependence of the temperature thereof, and the reflected light the color of which is thus dependent of the temperature of the membrane is transmitted to the control unit by fiber 34B for processing in the control unit and indication of the temperature of the membrane.

The temperature of membrane 21 also can be measured by other techniques known per se. E.g. a thermistor, resistor or a thermoelement can be integrated with the membrane.

Socket 20 also forms axial passages 36, FIG 6 and 9, which are connected to a vacuum pump in control unit 10 by conduits in hose 12 in order to provide a suction force on the surface against which the membrane is applied in order to keep the tissue thereof attracted against the membrane during operation of the apparatus.

Socket 26 can be screwed on bushing 18 for supplementary adjustment of the distance between the body surface to which the membrane 21 is applied, and the crystal 28 as illustrated in FIG 10. By this adjustment the relative position of socket 20 and crystal 28 is changed. As an alternative, means can be provided for displacement of crystal 28 in relation to socket 20 which in that case is fixedly mounted.

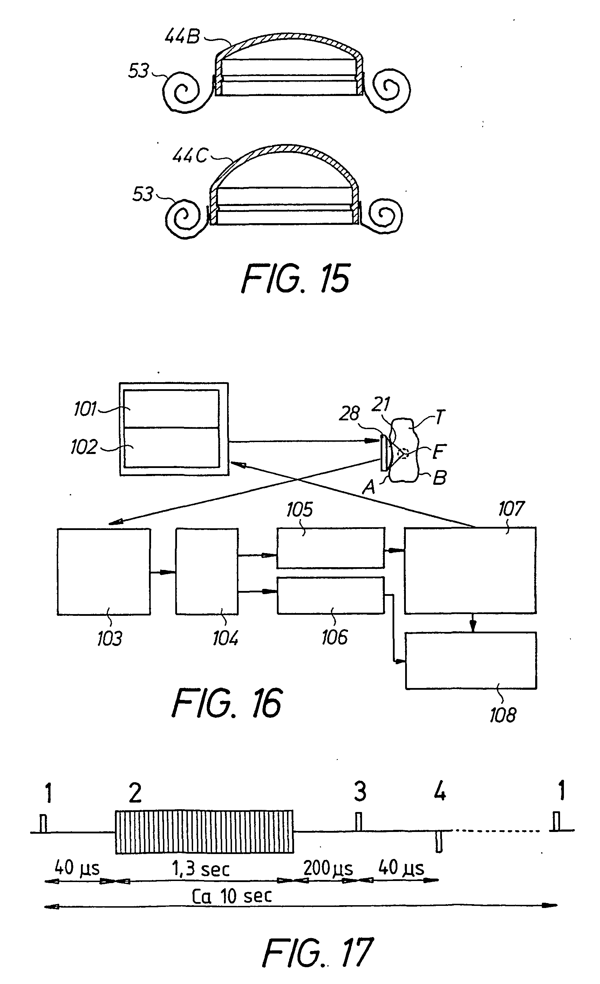

Referring to FIG 16 the control unit 10 comprises a transmitter 101 for generating diagnostic ultrasound energy (low intense) which is transmitted by the crystal (transducer) 28, and a transmitter 102 for generating therapeutic (high intense) ultrasound energy which is also transmitted by the crystal 28. The two transmitter curcuits can also be constructed as a single circuit. By means of the apparatus described the ultrasound energy is transmitted to tissue T to be treated from the crystal via the membrane 21 which is applied against an outside surface of the tissue. A receiver 103 including a wideband amplifier with controlled amplification is provided for receiving and amplifying ultrasound echo signals. The receiver 103 is connected to a analogue/digital converter 104 for converting signals received by the receiver from analogue form to digital form in order to facilitate subsequent signal processing. Output signals from the receiver are transmitted via the converter to an analyzer 105 and to a calculator 106. The analyzer 105 can be an FFT (fast Fourier transform) analyzer or a Doppler analyzer or correlating echoes from negative and positive transmitted ultrasound pulses. A single analyzer of one or a combination of the types mentioned can be provided. The output signal from the analyzer (or each analyzer) is transferred to a complex comparing circuit here called "a comparator" 107 wherein the signal is compared with a reference earlier stored. The comparator 107 is operatively connected with the transmitter 102. When a comparison indicates that the input signal equals a pre-set reference value the comparator shuts of the transmitter 102. A display 108 is connected to the calculator 106 and the comparator 107.

When the apparatus described is to be used for treatment of a patient the membrane 21 of the instrument 11 is applied against an outside front surface A of the tissue T. By means of diagnostic ultrasound signal pulses generated by the transmitter 101 and transmitted by the crystal 28 via the membrane 21 ultrasound echoes generated by ultrasound energy being reflected at the front and back surfaces A and B, respectively, of the tissue are received by the receiver 103 and are processed in the calculator 106 in order to determine the thickness of the tissue T. The echoes are also transmitted to the comparator 107 via an analyzer 105 of the FFT type for analysis of harmonics in the echo signals or to an analyzer 105 of the Doppler type for analysis of "movements" in the target area, or analysis of echoes from transmitted positive and negative pulses, or to a combination of analyzers of one and the other type, respective, and the output signal(s) from the analyzer(s) is received by the comparator 107.

With reference to FIG 17 which illustrates diagrammatically a typical sequence for effecting a non-invasive ultrasonic medical treatment according to the invention the several steps being marked on a time axis. Initially the thickness of the tissue between surfaces A and B is defined between positions 1 and 2. Echoes are received when the ultrasound passes through the front surface A and when the ultrasound passes through the back surface B. The distance between the surfaces is calculated in the calculator 106 on the basis of the time period between the echoes and the frequency of the ultrasound. The target area F to be treated usually is located substantially midway between the first and second surfaces. On the basis of the result of the measurement the distance between the crystal 28 and the membrane 21 applied against the tissue surface A is now adjusted in order to focus ultrasound energy emitted by the crystal 28 on the target area F located centrally in the tissue. This can be done by adjusting the pressure of the circulating fluid in order to expand the membrane 21 more or less and/or by adjusting the relative axial position of socket 26 and crystal 28. The pressure of the fluid is adjusted on control unit 10. Then, therapeutic ultrasound energy generated by the transmitter 102 is transmitted from the crystal 28 via membrane 21 and is focused on the target area for treatment of the tissue in said area.

Parameters of the treatment such as ultrasound intensity, temperature of the circulating fluid, etc are set on control unit 10.

Therapeutic ultrasound pulses are emitted from the apparatus for about 1.3 seconds and then there is a pause for a period of 8.7 seconds. This can also be scaled down by approximately a factor of 10. During the pause the result of the treatment is checked by using backscattered echoes from both the therapeutic and the diagnostic ultrasound pulse between end of pulse 2 and after the "analyzing-pulses" positions 3 and 4. Depending on the result the non-invasive treatment is repeated according to the procedure described for 1 to 10 minutes until the desired amount of fibrous tissue in the target area has been developed which is indicated by the comparison made in the comparator. When the signal received by the comparator 107 equals a preset value which indicates that the desired amount of fibrous tissue has been developed by the treatment by means of therapeutic ultrasound, the transmitter 102 is shut off by a signal emitted by the comparator.

A switch 38 is provided on handle 13 of instrument 11 for turning the apparatus on and off, and thus the therapeutic treatment can be interrupted at any time according to the judgement of the operator. Also light emitting diodes 39 are provided on the handle to indicate different phases of the treatment effected by means of the apparatus.

Socket 20 including membrane 21 and flange 22 with collar 23 which during the treatment performed by means of the apparatus come into contact with the patient, should be constructed as an exchangeable unit for either one way use to be discarded after each use, or for sterilization after each use said unit being detached from the instrument at snap attachment 24, 28. The remainder of the instrument which does not contact the patient should be constructed for multiple use.

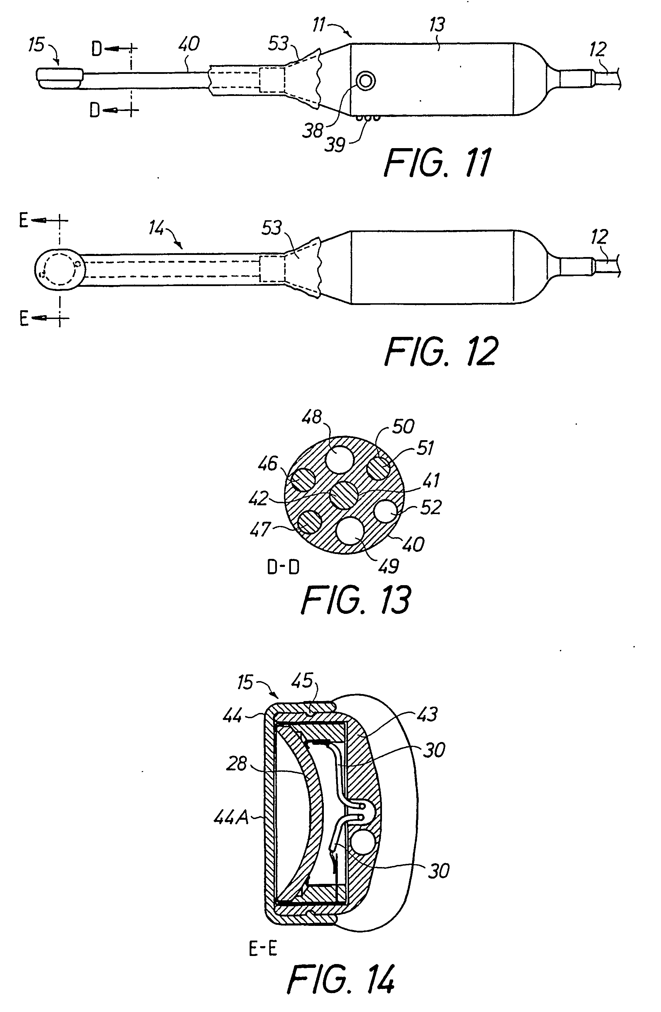

Referring now to FIGS 11 to 15 in the drawings the instrument 11 disclosed therein comprises a handle 13 provided with switch 38 and indicators 39 and adapted to be connected to the control unit 10 by hose 12. In this case the transducer head 15 is not facing axially from the end of the stem 14 but in the transverse direction thereof. The stem comprises a multiple lumen flexible hose 40 of silicone rubber receiving in a central lumen 41 thereof a metal wire 42 which imparts to the flexible stem the ability to maintain a shape which has been imparted to it.

The transducer head comprises a bottom element 43 which is permanently attached to the hose 40, and a cover element 44 which is connected by a snap connection 45 to the bottom element and forms together with the bottom element a sealed space enclosing the ultrasound crystal 28. Element 44 in this case is a substantially rigid element forming a plane surface 44A to be applied against a body surface, and thus does not allow the focusing of the emitted ultrasound energy to be adjusted in the manner described with reference to membrane 21. However, such adjustment can be effected by attaching to the bottom element cover elements 44 of different axial lengths. It is also possible to replace the element 44 forming a plane surface 44a by an element forming a convex surface 44B or an element forming a semi-spherical surface 44C, FIG 15. The wires 30 for connecting the crystal with the control unit are extended through lumens 46 and 47 in hose 40. Lumens 48 and 49 form passages for supplying cooling fluid to the crystal space and draining cooling fluid therefrom. A lumen 50 receives a temperature sensor 51, and a lumen 52 forms a suction passage for the purpose mentioned above.

Cover element 44 is sealed to a flexible tubular sheath 53 which is extended over the hose 40 forming the stem of the instrument. The cover element and the sheath should form a unit for one way use to be detached at snap connection 45 and be discarded after each treatment of a patient. Said unit prevents hose 40 and bottom element 43 including details mounted therein, from contacting the human body during operation of the apparatus.

Preferred embodiments have been described in order to illustrate the invention but it is obvious to the man skilled in the art that these embodiments are examples only and that modifications thereof can be made without departing from the scope of the invention as defined in the claims.

## Claims

1. An apparatus for high energetic ultrasonic treatment of tissue in a target area inside a living body from an outside surface or a body cavity of the living body in order to achieve a fibrous tissue development, comprising an ultrasound generator (101, 102), a device (21) to be applied against the skin or mucous membrane at the site of treatment, and a transducer (28) connected with the ultrasound generator to emit generated ultrasound energy through said device, wherein the ultrasound generator is designed to generate pulsed therapeutic and diagnostic ultrasound energy to be emitted by the transducer, and wherein means are provided for cooling a contact surface of said device to be engaged with the skin or mucuous membrane, said device being made as an exchangeable part forming a heat exchange element between said device and the tissue, **characterized in that** a comparator (107) is provided for comparing echoes of diagnostic ultrasound energy from treated tissue in the target area with backscattered signals of either diagnostic or therapeutic ultrasound energy from untreated tissue to control the development of fibrous tissue in the target area.

2. The apparatus as in claim 1 wherein the comparator (107) is operatively connected with the ultrasound generator (101, 102) to interrupt the transmission of therapeutic ultrasound energy when backscattered signals from untreated tissue equal a reference signal.

3. The apparatus as in claim 1 or 2 further comprising a calculator (106) for calculating the thickness of the tissue between two surfaces (A, B) by means of echoes of diagnostic ultrasound energy received at said surfaces.

## Patentansprüche

1. Gerät zur hochenergetischen Ultraschallbehandlung eines Gewebes in einem Zielgebiet in einem lebenden Körper von einer äußeren Oberfläche oder einem Körperhohlraum des lebenden Körpers aus, um eine Fasergewebebildung zu erreichen, umfassend einen Ultraschallgenerator (101, 102), eine Vorrichtung (21), zur Verwendung auf der Haut oder Schleimhaut an der Behandlungsstelle, sowie einen Wandler (28), welcher mit dem Ultraschallgenerator verbunden ist, um Ultraschallenergie durch die genannte Vorrichtung abzugeben, wobei der Ultraschallgenerator konstruiert ist, um gepulste therapeutische und diagnostische Ultraschallenergie zur Abgabe durch den Wandler zu erzeugen, wobei Mittel zum Kühlen einer Kontaktoberfläche der genannten Vorrichtung, zum Eingriff mit der Haut oder Schleimhaut, vorgesehen sind, wobei die genannt Vorrichtung als auswechselbares Teil ausgebildet ist, welches ein Wärmeaustauschelement zwischen der Vorrichtung und dem Gewebe bildet, **dadurch gekennzeichnet, dass** ein Komparator (107) zum Vergleichen von Echos von diagnostischer Ultraschallenergie von behandeltem Gewebe im Zielgebiet mit rückgestreuten Signalen von entweder therapeutischer oder diagnostischer Ultraschallenergie von unbehandeltem Gewebe vorgesehen ist, um die Bildung von Fasergewebe im Zielgebiet zu regeln.

2. Gerät gemäß Anspruch 1, wobei der Komparator (107) operativ mit dem Ultraschallgenerator (101, 102) verbunden ist, um die Aussendung von therapeutischer Ultraschallenergie abzubrechen, wenn rückgestreute Signale von unbehandeltem Gewebe gleich einem Referenzsignal sind.

3. Gerät gemäß Anspruch 1 oder 2, des Weiteren umfassend eine Recheneinheit (106) zum Berechnen der Gewebedicke zwischen zwei Oberflächen (A, B) mithilfe Echos von diagnostischer Ultraschallenergie, welche an den genannten Oberflächen empfangen wird.

## Revendications

1. Appareil de traitement de tissu par ultrasons à haute énergie, dans une zone cible située à l'intérieur d'un corps vivant, depuis une surface extérieure ou une cavité corporelle du corps vivant afin d'obtenir un développement de tissu fibreux, comportant un générateur d'ultrasons (101, 102), un dispositif (21) à appliquer contre la peau ou une membrane muqueuse sur le site du traitement et un transducteur (28) connecté au générateur d'ultrasons pour émettre une énergie d'ultrasons générée à travers ledit dispositif, dans lequel le générateur d'ultrasons est destiné à générer une énergie d'ultrasons pulsée, thérapeutique et de diagnostic, à émettre par le transducteur et dans lequel il est prévu des moyens pour refroidir une surface de contact dudit dispositif à destinée à entrer en contact avec la peau ou une membrane muqueuse, ledit dispositif étant constitué par une pièce interchangeable formant un élément d'échange de chaleur entre ledit dispositif et le tissu, **caractérisé en ce qu'**il est prévu un comparateur (107) pour comparer les échos de l'énergie des ultrasons de diagnostic ou thérapeutiques provenant du tissu traité dans la zone cible avec les signaux rétro diffusés de l'énergie des ultrasons thérapeutiques ou de diagnostic provenant du tissu non traité pour contrôler le développement de tissu fibreux dans la zone cible.

2. Appareil selon la revendication 1, dans lequel le comparateur (107) est fonctionnellement connecté avec le générateur d'ultrasons (101, 102) pour interrompre la transmission de l'énergie thérapeutique des ultrasons lorsque les signaux rétro diffusés provenant du tissu non traité sont égaux à un signal de référence.

3. Appareil selon la revendication 1 ou 2, comportant en outre un calculateur (106) pour calculer l'épaisseur du tissu entre deux surfaces (A, B) au moyen des échos de l'énergie de diagnostic des ultrasons reçus sur ces surfaces.
